(19) Europäisches Patentamt
European Patent Office
Office européen des brevets

(11) Veröffentlichungsnummer: **0 056 616**
**B1**

(12) # EUROPÄISCHE PATENTSCHRIFT

(45) Veröffentlichungstag der Patentschrift: **21.08.85**

(21) Anmeldenummer: **82100203.7**

(22) Anmeldetag: **13.01.82**

(51) Int. Cl.⁴: **C 07 C 87/459,** C 07 D 295/02, A 61 K 31/645 // C07C23/18, C07C35/37, C07C57/38, C07C69/616, C07D295/18

(54) **Cycloheptenderivate, Verfahren zu ihrer Herstellung, sowie diese enthaltende Arzneimittel.**

(30) Priorität: **16.01.81 CH 274/81**

(43) Veröffentlichungstag der Anmeldung: **28.07.82 Patentblatt 82/30**

(45) Bekanntmachung des Hinweises auf die Patenterteilung: **21.08.85 Patentblatt 85/34**

(84) Benannte Vertragsstaaten: **BE CH DE FR GB IT LI NL SE**

(56) Entgegenhaltungen:
**DE - A - 1 922 280**
**GB - A - 1 017 696**
**US - A - 3 409 640**

**HELVETICA CHIMICA ACTA, Band 53, Heft 5, 1970 BASEL (CH) P. DOSTERT et al.: "Nr. 105. Synthèse par réaction de Friedel-Crafts d'analogues partiellement saturés de l'amitriptyline" Seiten 882-897**
**HELVETICA CHIMICA ACTA, Band 53, Heft 5, 1970 BASEL (CH) P. DOSTERT et al.: "Nr. 106. Synthèse d'analogues partiellement saturés de l'amitriptyline à partir d'ortho-tolunitriles" Seiten 897-904**
**CHEMICAL ABSTRACTS; Band 72, 1970 Zusammenfassung: 1938k, Seite 172 COLUMBUS, OHIO (US) W. LIPPMANN: "Blockade of norepinephrine uptake and other activities of 5-(3-dimethylaminopropyl)dibenzo (a,d)(1,4)cycloheptadiene hydrochloride (AY-8794) and structurally related compounds"**
**CHEMICAL ABSTRACTS 8th COLLECTIF INDEX, 1967-71 SUBJECT INDEX Seite 9841S, Spalte 2**

(73) Patentinhaber: **F. HOFFMANN-LA ROCHE & CO. Aktiengesellschaft, CH-4002 Basel (CH)**

(72) Erfinder: **Aschwanden, Werner, Grellingerstrasse 4, CH-4107 Ettingen (CH)**
Erfinder: **Branca, Quirico, Dr., Lenzgasse 2, CH-4056 Basel (CH)**
Erfinder: **Kyburz, Emilio, Dr., Unterer Rebbergweg 127, CH-4153 Reinach (CH)**
Erfinder: **Pfister, Rudolf, Dr., Neubadstrasse 128, CH-4054 Basel (CH)**

(74) Vertreter: **Lederer, Franz, Dr. et al, Patentanwälte Dr. Lederer Franz Meyer-Roxlau Reiner F. Lucile-Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

**Beschreibung**

Die vorliegende Erfindung betrifft Cycloheptenderivate. Im speziellen betrifft sie Cycloheptenderivate der allgemeinen Formel

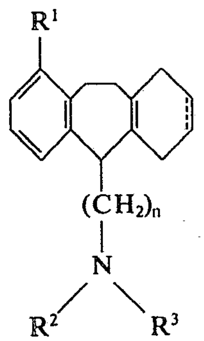

(I)

worin $R^1$ niederes Alkyl, die gestrichelte Linie eine fakultative Bindung und n die Zahl 1, 2 oder 3 bedeuten, und entweder $R^2$ Wasserstoff oder niederes Alkyl und $R^3$ niederes Alkyl bedeuten, oder $R^2$ und $R^3$ zusammen mit dem Stickstoffatom einen 5- oder 6-gliedrigen Heterocyclus bedeuten, und pharmazeutisch annehmbare Säureadditionssalze davon.

In Helv. 53, 882—897 (1970) und Helv. 53, 897—904 (1970) werden partiell gesättigte Analoga des Amitriptylins und ihre antidepressiven Eigenschaften beschrieben. In der Deutschen Offenlegungsschrift 1 922 280 werden unter anderem 5H-Divbenzo-[a,d]cycloheptene mit einer basischen Seitenkette in 5-Stellung und einer Methylgruppe in 1-Stellung beschrieben, welche insbesondere ausgeprägte antidepressive Eigenschaften besitzen.

Diese Verbindungen sind neu; sie zeichnen sich durch wertvolle therapheutische Eigenschaften aus und können demnach bei der Bekämpfung bzw. Verhütung verwendet werden.

Gegenstand der vorliegenden Erfindung sind Verbindungen der allgemeinen Formel I und ihre pharmazeutisch annehmbaren Säureadditionssalze als solche und als pharmazeutische Wirkstoffe, die Herstellung dieser Verbindungen und Arzneimittel, enthaltend eine solche Verbindung.

Der Ausdruck »niederes Alkyl« bezeichnet geradkettige oder verzweigte, gesättigte Kohlenwasserstoffreste mit höchstens 7, vorzugsweise höchstens 4 Kohlenstoffatomen, wie Methyl, Äthyl, n-Propyl, Isopropyl, n-Butyl, s-Butyl, t-Butyl, Isobutyl und dergleichen. Der Ausdruck »5- oder 6-gliedriger Heterocyclus« umfaßt gegebenenfalls durch niederes Alkyl substituierte 1-Pyrrolidinyl-, 1-Piperidinyl-, 4-Morpholinyl-, 1-Piperazinyl-, 3-Thiazolidinylreste und dergleichen.

Der Ausdruck »pharmazeutisch annehmbare Säureadditionssalze« umfaßt pharmazeutisch annehmbare Salze von Verbindungen der allgemeinen Formel I sowohl mit anorganischen Säuren, wie Salzsäure, Bromwasserstoff, Phosphorsäure, Schwefelsäure und dergleichen, als auch mit organischen Säuren, wie Maleinsäure, Citronensäure, Essigsäure, Bernsteinsäure, Apfelsäure, Weinsäure, Camphersulfonsäure, Mandelsäure, Fumarsäure, Methansulfonsäure, p-Toluolsulfonsäure und dergleichen. Die Herstellung der pharmazeutisch annehmbaren Säureadditionssalze erfolgt nach an sich bekannten und jedem Fachmann geläufigen Methoden.

Die Verbindungen der allgemeinen Formel I besitzen ein asymmetrisches Kohlenstoffatom; die vorliegende Erfindung umfaßt sowohl die optisch einheitlichen Formen als auch Gemische davon (insbesondere die Racemate). Die Spaltung der Racemate kann nach an sich bekannten Methoden durchgeführt werden, beispielsweise durch fraktionierte Kristallisation eines Säureadditionssalzes einer Verbindung der Formel I mit einer optisch aktiven Säure (z. B. mit Weinsäure, Camphersulfonsäure, Mandelsäure und dergleichen). Die optisch einheitlichen Formen können aber auch erhalten werden, indem man in den nachfolgend beschriebenen Verfahrensvarianten a), b) und d) optisch einheitliche Ausgangsstoffe verwendet.

Der weiter unten verwendete Ausdruck »Abgangsgruppe« umfaßt Halogenatome, wie Chlor, Brom und Jod, Sulfonsäurereste wie Methansulfonyloxy, p-Toluolsulfonyloxy, p-Brombenzolsulfonyloxy, Benzolsulfonyloxy und dergleichen, und andere äquivalente Abgangsgruppen.

Die bevorzugte Bedeutungsmöglichkeit von $R^1$ ist Methyl. Der Rest $-NR^2R^3$ bedeutet vorzugsweise Methylamino, Dimethylamino, Diäthylamino, 1-Pyrrolidinyl oder 1-Piperidinyl.

Eine im Rahmen der vorliegenden Erfindung ganz besonders bevorzugte Verbindung ist das 4,5,10,11-Tetrahydro-N,N,9-trimethyl-1H-dibenzo[a,d]cyclohepten-5-propylamin.

Weitere besonders bevorzugte, von der allgemeinen Formel I umfaßte Verbindungen sind:

2,3,4,5,10,11-Hexahydro-N,N,9-trimethyl-1H-dibenzo[a,d]cyclohepten-5-propylamin und
1-[2-(4,5,10,11-Tetrahydro-9-methyl-1H-dibenzo[a,d]cyclohepten-5-yl)äthyl]-pyrrolidin.

Die Verbindungen der allgemeinen Formel I und ihre pharmazeutisch annehmbaren Säureadditionssalze können erfindungsgemäß dadurch hergestellt werden, daß man

a) in einer Verbindung der allgemeinen Formel

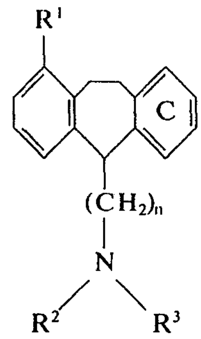

(II)

worin $R^1$, $R^2$, $R^3$ und n obige Bedeutung besitzen, den mit C bezeichneten aromatischen Ring 1,4-reduziert, oder

b) in einer Verbindung der allgemeinen Formel

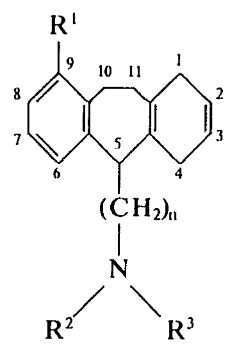

(Ia)

$R^1$, $R^2$, $R^3$ und n obige Bedeutung besitzen, die 2,3-Doppelbindung hydriert, oder

c) eine Verbindung der allgemeinen Formel

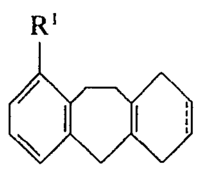

(III)

worin $R^1$ und die gestrichelte Linie obige Bedeutung besitzen, in Gegenwart einer starken Base mit einer Verbindung der allgemeinen Formel

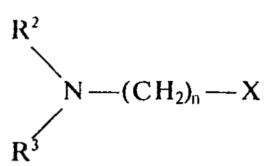

(IV)

worin $R^2$, $R^3$ und n obige Bedeutung besitzen, und X eine Abgangsgruppe bedeutet, umsetzt, oder

d) eine Verbindung der allgemeinen Formel

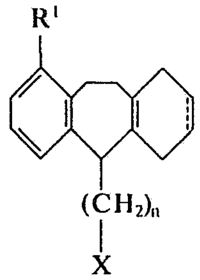

(V)

worin $R^1$, die gestrichelte Linie, n und X obige Bedeutung besitzen, mit einem Amin der allgemeinen Formel

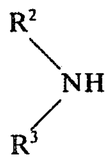

(VI)

worin $R^2$ und $R^3$ obige Bedeutung besitzen, umsetzt, und

e) eine erhaltene Verbindung der allgemeinen Formel I als freie Base oder als pharmazeutisch annehmbares Säureadditionssalz isoliert.

Nach Variante a) des erfindungsgemäßen Verfahrens kann eine Verbindung der allgemeinen Formel I, worin die gestrichelte Linie eine zusätzliche Bindung bedeutet, hergestellt werden, indem man in einer Verbindung der allgemeinen Formel II den mit C bezeichneten aromatischen Ring 1,4-reduziert. Als Reduktionsmittel kann man beispielsweise ein Alkalimetall, wie Lithium, Natrium oder Kalium, verwenden, wobei man als Lösungsmittel flüssiges Ammoniak oder ein für solche Reduktionen geeignetes Amin, wie Methylamin, Äthylamin, Dimethylamin oder dergleichen, einsetzt. Zweckmäßigerweise arbeitet man in Gegenwart eines Lösungsvermittlers und/oder eines Protonendonators. Geeignete Lösungsvermittler sind beispielsweise Äther, wie Diäthyläther, t-Butylmethyläther, Tetrahydrofuran, Dioxan, Äthylenglykoldimethyläther, Diglyme und dergleichen. Als Protonendonatoren kommen in erster Linie Alkohole, wie Methanol, Äthanol, Propanol, Isopropanol, Butanol, t-Butanol, 1,1-Dimethylpropanol, Äthylenglykolmonomethyläther, Propylenglykolmonomethyläther und dergleichen, in Frage. Die Reaktionstemperatur liegt je nach verwendetem Lösungsmittel in einem Bereich von etwa −50° C bis Siedetemperatur des Reaktionsgemisches.

Die 1,4-Reduktion kann beispielsweise so durchgeführt werden, daß man eine Lösung einer Verbindung der allgemeinen Formel II, in einem der obengenannten Lösungsmitteln, vorzugsweise in siedendem Ammoniak, oder gegebenenfalls in einer Mischung aus dem Lösungsmittel, dem Lösungsvermittler, vorzugsweise trockenem Tetrahydrofuran, und/oder dem Protonendonator, vorzugsweise trockenem t-Butanol oder Äthanol, vorbereitet, und diese Lösung mit dem Alkalimetall, vorzugsweise Lithium oder Natrium versetzt.

Man kann aber ohne weiteres auch umgekehrt verfahren, d. h. man kann eine Lösung des Alkalimetalls im Lösungsmittel vorbereiten, und diese mit einer Verbindung der allgemeinen Formel II, oder gegebenenfalls mit einer Lösung dieser Verbindung im Lösungsvermittler und/oder Protonendonator versetzen.

Eine weitere Ausführungsform besteht darin, daß man den Protonendonator erst nach beendeter 1,4-Reduktion dem Reaktionsgemisch zugibt. In diesem Fall eignen sich als Protonendonatoren auch saure Ammoniumsalze, wie Ammoniumchlorid und dergleichen.

Schließlich kann die gewünschte 1,4-Reduktion auch elektrochemisch durchgeführt werden. Die elektrochemische 1,4-Reduktion kann in einer ungeteilten oder in einer geteilten Zelle durchgeführt werden, wobei die ungeteilte Zelle bevorzugt wird. Das Kathodenmaterial ist nicht kritisch, und man kann demnach Platin, Graphit, Quecksilber, Blei, Nickel, Aluminium und dergleichen verwenden. Als Kathodenmaterial verwendet man vorzugsweise Platin. Das bevorzugte Anodenmaterial ist Platin, wobei auch Blei oder Graphit oder andere nicht korrodierende Werkstoffe verwendet werden können. Als Lösungsmittel kommen Amine, wie Methylamin, Propylamin und Äthylendiamin, oder dergleichen in Frage. Gegebenenfalls können auch Lösungsvermittler, wie Tetrahydrofuran und Diäthylenglykoldimethyläther, und/oder Protonendonatoren, wie Äthanol und t-Butanol, eingesetzt werden. Für den vorliegenden Verfahrensaspekt geeignete Leitsalze sind beispielsweise Lithiumchlorid, Natriumchlorid, Tetrabutylammoniumchlorid und dergleichen. Die Reaktionstemperatur ist nicht kritisch, man kann

demnach in Abhängigkeit vom eingesetzten Lösungsmittel in einem Temperaturbereich von etwa −20° C bis etwa 100° C arbeiten.

In einer besonders bevorzugten Ausführungsform verwendet man Methylamin als Lösungsmittel, Lithiumchlorid als Leitsalz und Platin als Anoden- und Kathodenmaterial und arbeitet bei einer Temperatur von etwa −10° C.

Nach Variante b) des erfindungsgemäßen Verfahrens können Verbindungen der allgemeinen Formel I, worin die gestrichelte Linie keine zusätzliche Bindung bedeutet, hergestellt werden, indem man in einer Verbindung der allgemeinen Formel Ia die 2,3-Doppelbindung hydriert. Diese Reaktion kann nach an sich bekannten und jedem Fachmann geläufigen Methoden durchgeführt werden. In einer bevorzugten Ausführungsform verwendet man elementaren Wasserstoff als Reduktionsmittel und arbeitet in Gegenwart eines in solchen Fällen üblichen Katalysators, wie Palladium/Kohle, Raney-Nikkel, Platinoxid, Palladium/Aluminiumoxid und dergleichen, in einem unter den Reaktionsbedingungen inerten organischen Lösungsmittel, beispielsweise in einem Alkohol, wie Methanol, Äthanol, usw., in einem Äther, wie Diäthyläther, Tetrahydrofuran, Dioxan, usw., in Essigester, Essigsäure und dergleichen. Je nach Reaktivität des Katalysators arbeitet man bei Drucken von etwa Normaldruck bis etwa 300 bar und bei Temperaturen von etwas Raumtemperatur bis etwa 300° C.

Nach Variante c) des erfindungsgemäßen Verfahrens kann eine Verbindung der allgemeinen Formel I hergestellt werden, indem man eine Verbindung der allgemeinen Formel III in Gegenwart einer starken Base mit einer Verbindung der allgemeinen Formel IV umsetzt. Zweckmäßigerweise wird die Verbindung der allgemeinen Formel III in einem inerten organischen Lösungsmittel, beispielsweise in einem Äther, wie Tetrahydrofuran, Dioxan, Diäthyläther, Dimethoxyäthan, Diglyme, t-Butylmethyläther oder dergleichen, oder in einer Mischung davon mit Alkanen, wie z. B. Pentan, Hexan und Heptan, mit einer starken Base, beispielsweise mit einer Alkyl- oder Aryllithiumverbindung, wie n-Butyllithium, Methyllithium und Phenyllithium, oder mit einem Alkalimetallamid, wie Lithiumdiisopropylamid und Natriumamid, oder mit Natriumhydrid oder dergleichen, in das entsprechende Anion übergeführt, und dieses mit einer Verbindung der allgemeinen Formel IV umgesetzt. Je nach eingesetzter Base kann man bei Temperaturen von etwa −70° C bis etwa Raumtemperatur arbeiten.

Nach Variante d) des erfindungsgemäßen Verfahrens kann eine Verbindung der allgemeinen Formel I hergestellt werden, indem man eine Verbindung der allgemeinen Formel V mit einem Amin der allgemeinen Formel VI umsetzt. Diese Reaktion erfolgt zweckmäßigerweise in einem inerten organischen Lösungsmittel in Gegenwart eines säurebindenden Mittels. Für den vorliegenden Verfahrensaspekt geeignete Lösungsmittel sind beispielsweise Äther, wie Diäthyläther, t-Butylmethyläther, Tetrahydrofuran, Äthylenglykoldimethyläther und dergleichen, Alkohole, wie Äthanol, Äthylenglykol und dergleichen, oder überschüssige Amin der allgemeinen Formel VI. Als säurebindende Mittel kommen anorganische Basen, wie Kalium- und Natriumcarbonat oder dergleichen, oder organische Basen, wie Triäthylamin, Chinuclidin oder dergleichen, oder überschüssiges Amin der allgemeinen Formel VI in Frage. In einer bevorzugten Ausführungsform verwendet man überschüssiges Amin der allgemeinen Formel VI als Lösungsmittel und gleichzeitig als säurebindendes Mittel. Die Reaktionstemperatur kann in einem Bereich von etwa 0° C bis Siedetemperatur des Reaktionsgemisches variieren und ist natürlich abhängig von der Reaktivität der mit X bezeichneten Abgangsgruppe. Eine ganz besonders bevorzugte Abgangsgruppe ist die p-Toluolsulfonyloxygruppe.

Erfindungsgemäß wird eine erhaltene Verbindung der allgemeinen Formel I als freie Base oder als pharmazeutisch annehmbares Säureadditionssalz isoliert. Die Isolierung einer freien Base oder eines pharmazeutisch annehmbaren Säureadditionssalzes davon erfolgt nach an sich bekannten und jedem Fachmann geläufigen Methoden, beispielsweise durch Extraktions- oder Filtrationstechniken, durch gegebenenfalls fraktionierte Kristallisation, durch chromatographische Methoden, wie Gaschromatographie und Hochdruck-Flüssigkeits-Chromatographie, der durch Destillation, oder durch geeignete Kombination mehrerer dieser an sich bekannten Methoden.

Die als Ausgangsstoffe verwendeten Verbindungen, der allgemeinen Formel II gehören einer an sich bekannen Verbindungsklasse an; spezifisch nicht vorbeschriebene Vertreter dieser Verbindungsklasse können in Analogie zu den bekannten Vertreter hergestellt werden. Das weiter unten folgende Beispiel 3 enthält detaillierte Angaben betreffend die Herstellung von Verbindungen der allgemeinen Formel II.

Die als Ausgangsstoffe verwendeten Verbindungen der allgemeinen Formel III können beispielsweise hergestellt werden, indem man in einer Verbindung der allgemeinen Formel

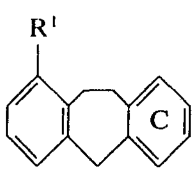

(VII)

worin $R^1$ obige Bedeutung besitzt, den mit C bezeichneten aromatischen Ring in Analogie zu Verfahrensvariante a) 1,4-reduziert und gegebenenfalls in einer erhaltenen Verbindung der allgemeinen Formel

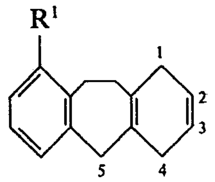

(IIIa)

worin $R^1$ obige Bedeutung besitzt, die 2,3-Doppelbindung in Analogie zu Verfahrensvariante b) hydriert.

Die Verbindungen der allgemeinen Formel VII sind bekannt oder können nach an sich bekannten und jedem Fachmann geläufigen Methoden hergestellt werden.

Ausgehend von Verbindungen der allgemeinen Formel

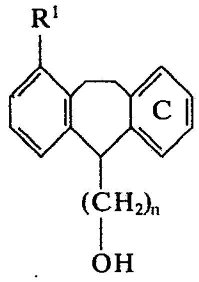

(VIII)

worin $R^1$ und n obige Bedeutung besitzen, die einer an sich bekannten Stoffklasse angehören und die nach bekannten und jedem Fachmann geläufigen Methoden hergestellt werden können, können die als Ausgangsstoffe verwendeten Verbindungen der allgemeinen Formel V dadurch gekennzeichnet werden, daß man in einer Verbindung der allgemeinen Formel VIII den mit C bezeichneten aromatischen Ring in Analogie zu Verfahrensvariante a) 1,4-reduziert, und gegebenenfalls im erhaltenen Produkt die 2,3-Doppelbindung in Analogie zu Verfahrensvariante b) hydriert.

Eine so erhaltene Verbindung der allgemeinen Formel

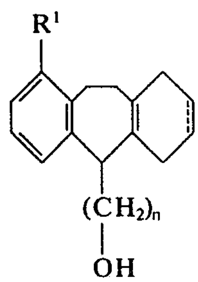

(IX)

worin $R^1$, die gestrichelte Linie und n obige Bedeutung besitzen, kann nun nach an sich bekannten und jedem Fachmann geläufigen Methoden in eine Verbindung der allgemeinen Formel V übergeführt werden. Beispielsweise kann man in einer Verbindung der allgemeinen Formel IX die Hydroxygruppe mit einer organischen Sulfonsäure verestern, und zwar durch Behandeln mit einem reaktiven Sulfonsäurederivat, wie Methansulfonsäurechlorid, Benzolsulfonsäurechlorid, p-Toluolsulfonsäurechlorid, p-Brombenzolsulfonsäurechlorid oder dergleichen, und, sofern als Abgangsgruppe ein Halogenatom gewünscht wird, den Sulfonsäurerest in an sich bekannter Weise durch ein Halogenatom, wie Chlor, Brom oder Jod, ersetzen. Die Halogenide können aber auch direkt aus den entsprechenden Alkoholen der allgemeinen Formel IX erhalten werden, beispielsweise durch Umsetzen mit einem Halogenierungsmittel, wie Thionylchlorid, Phosphoroxychlorid, Phosphorpentachlorid, Tetrabromkohlenstoff/Triphenylphosphin, Jod/roter Phosphor oder dergleichen.

Die Verbindungen der allgemeinen Formeln III und V sind neu.

Überraschenderweise hat es sich gezeigt, daß die erfindungsgemäßen Produkte die $H_1$-Wirkung des Histamins zu hemmen vermögen. Die Verbindungen der allgemeinen Formel I und ihre pharmazeutisch annehmbaren Säureadditionssalze sind somit wertvolle Histamin-$H_1$ antagonistische Wirkstoffe und eignen sich insbesondere zur Bekämpfung bzw. Verhütung von allergischen Reaktionen, wie beispielsweise Urtikaria, Heuschnupfen, Anaphylaxie und Arzneimittelüberempfindlichkeit. Diese Histamin-$H_1$ antagonistischen Eigenschaften können wie nachfolgend beschrieben an männlichen und weiblichen, 240 bis 300 g schweren Meerschweinchen (SPF, Füllinsdorf) ermittelt werden:

Den Versuchstieren (10 pro Dosis) wird während 24 Stunden den vor Versuchsbeginn das Futter entzogen, wobei Wasser ad libitum erhältlich ist. Eine Stunde nach oraler Verabreichung einer Lösung der Testsubstanz (10 ml/kg) erhalten die Versuchstiere eine letale Dosis von Histamin-dihydrochlorid (10 mg/kg s. c.). Ungeschützte Tiere, d. h. nur mit Histamin-dihydrochlorid behandelte Tiere, sterben innerhalb einer Stunde. Nach Auszählen der überlebenden, geschützten Tiere wird die $ED_{50}$ nach der Probitmethode ermittelt. Die $ED_{50}$ ist diejenige Dosis, die notwendig ist, um 50% der mit der Testsubstanz behandelten Tiere vor dem Tod zu schützen.

Die nachfolgende Tabelle enthält für zwei repräsentative Vertreter der durch die allgemeinen Formel I umfaßten Verbindungsklasse $ED_{50}$-Werte, sowie Angaben über deren akute Toxizität bei einmaliger oraler Verabreichung an Mäusen ($DL_{50}$ in mg/kg).

Tabelle

| Testverbindung | $ED_{50}$ in mg/kg p. o. | $DL_{50}$ in mg/kg p. o. |
|---|---|---|
| A | 0,4 | 600—1200 |
| B | 0,9 | 250—500 |

A: 4,5,10,11-Tetrahydro-N,N,9-trimethyl-1H-dibenzo[a,d]cyclohepten-5-propylamin;
B: 2,3,4,5,10,11-Hexahydro-N,N,9-trimethyl-1H-dibenzo[a,d]cyclohepten-5-propylamin.

Die Verbindungen der allgemeinen Formel I und ihre pharmazeutisch annehmbare Säureadditionssalze können als Heilmittel, z. B. in Form pharmazeutischer Präparate, Verwendung finden. Die pharmazeutischen Präparate können oral, z. B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z. B. in Form von Suppositorien, oder parenteral, z. B. in Form von Injektionslösungen, erfolgen.

Zur Herstellung von pharmazeutischen Präparaten können die erfindungsgemäßen Produkte mit pharmazeutisch inerten, anorganischen oder organischen Trägern verarbeitet werden. Als solche Träger kann man für Tabletten, Lacktabletten, Dragées und Hartgelatinekapseln beispielsweise Lactose, Maisstärke oder Derivate davon, Talk, Stearinsäure oder deren Salze und dergleichen verwenden. Für Weichgelatinekapseln eignen sich als Träger beispielsweise pflanzliche Öle, Wachse, Fette, halbfeste und flüssige Polyole und dergleichen; je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Polyole, Saccharose, Invertzucker, Glukose und dergleichen. Für Injektionslösungen eignen sich als Träger beispielsweise Wasser, Alkohole, Polyole, Glyzerin, pflanzliche Öle und dergleichen. Für Suppositorien eignen sich als Träger beispielsweise natürliche oder gehärtete Öle, Wachse, Fette, halbflüssige oder flüssige Polyole und dergleichen.

Die pharmazeutischen Präparate können daneben noch Konservierungsmittel, Lösungsvermittler, Stabilisierungsmittel, Netzmittel, Emulgiermittel, Süßmittel, Färbemittel, Aromatisierungsmittel, Salze zur Veränderung des osmotischen Druckes, Puffer, Überzugsmittel oder Antioxidantien enthalten. Sie können auch noch andere therapeutisch wertvolle Stoffe enthalten.

Wie eingangs erwähnt sind Arzneimittel, enthaltend eine Verbindung der allgemeinen Formel I oder ein pharmazeutisch annehmbares Säureadditionssalz davon, ebenfalls Gegenstand der vorliegenden Erfindung. Ein Verfahren zur Herstellung solcher Arzneimittel, ist dadurch gekennzeichnet, daß man eine Verbindung der allgemeinen Formel I oder ein pharmazeutisch annehmbares Säureadditionssalz davon und gegebenenfalls einen oder mehrere andere therapeutisch wertvolle Stoffe in eine galenische Darreichungsform bringt. Wie eingangs erwähnt, kann man die Verbindungen der allgemeinen Formel I und ihre pharmazeutisch annehmbare Säureadditionssalze bei der Bekämpfung bzw. Verhütung von allergischen Reaktionen, wie Urtikaria, Heuschnupfen, Anaphylaxie und Arzneimittelüberempfindlichkeit verwenden. Die Dosierung kann dabei innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 10 mg bis 150 mg angemessen sein.

In den nachfolgenden Beispielen, welche die vorliegende Erfindung näher erläutern, ihren Umfang jedoch in keiner Weise beschränken sollen, sind sämtliche Temperaturen in Celsiusgraden angegeben.

Beispiel 1

Eine Lösung von 10,2 g 10,11-Dihydro-N,N,9-trimethyl-5H-dibenzo[a,d]cyclohepten-5-propylamin in 150 ml trockenem Tetrahydrofuran und 50 ml t-Butanol wird vorgelegt und mit 300 ml trockenem flüssigem Ammoniak versetzt. Über einen Zeitraum von 30 Minuten gibt man bei der Siedetemperatur 1,4 g Lithiumschnitzel hinzu. Nach Umschlag der dunkelblauen Färbung auf farblos versetzt man tropfenweise mit 20 ml Äthanol und entfernt das Ammoniak. Der Rückstand wird im Vakuum eingedampft und zwischen Diäthyläther und gesättigter Natriumchloridlösung verteilt. Die organische Phase wird mit gesättigter Natriumchloridlösung gewaschen, über Magnesiumsulfat getrocknet und eingedampft. Man erhält 4,5,10,11-Tetrahydro-N,N,9-trimethyl-1H-dibenzo[a,d]cyclohepten-5-propylamin, als gelbliches Öl.

Das entsprechende Maleinsäuresalz kristallisiert aus Aceton/Diäthyläther und hat einen Schmelzpunkt von 103—105°.

Beispiel 2

Eine Lösung von 2,4 g 4,5,10,11-Tetrahydro-N,N,9-trimethyl-1H-dibenzo[a,d]cyclohepten-5-propylamin in 100 ml Äthanol wird über 0,25 g 5-proz. Palladiumkohle bei Normaldruck hydriert. Nach Abfiltrieren des Katalysators wird das Reaktionsgemisch eingedampft, und die verbleibende rohe Base in 15 ml Aceton aufgenommen. Man versetzt zuerst mit einer Lösung von 0,975 g Malseinsäure in 10 ml Aceton und dann mit 125 ml Äther. Man läßt über Nacht im Eisschrank stehen und filtriert das auskristallisierte Material ab. Nach Umkristallisieren aus Aceton/Äther erhält man 2,3,4,5,10,11-Hexahydro-N,N,9-trimethyl-1H-dibenzo[a,d]cyclohepten-5-propylamin-maleinat vom Schmelzpunkt 114—117,5°.

Beispiel 3

a) Zu 212 g 10,11-Dihydro-1-methyl-5H-dibenzo[a,d]cyclohepten-5-on und 700 ml Dioxan gibt man eine Lösung von 72,5 g Natriumborhydrid in 150 ml Wasser und 300 ml Dioxan hinzu und rührt das erhaltene Gemisch während 16 Stunden bei 60°. Nach Eindampfen des Reaktionsgemisches wird der Rückstand zwischen 1000 ml Toluol und dreimal 500 ml Wasser verteilt. Die organische Phase wird mit Wasser gewaschen, über Magnesiumsulfat getrocknet und eingedampft. Man erhält nach Umkristallisieren aus Diäthyläther/Petroläther (tiefsiedend) 10,11-Dihydro-1-methyl-5H-dibenzo[a,d]cyclohepten-5-ol vom Schmelzpunkt 102—105°.

b) Eine Lösung von 207 g 10,11-Dihydro-1-methyl-5H-dibenzo[a,d]cyclohepten-5-ol in 700 ml Benzol wird über einen Zeitraum von 40 Minuten mit 344 ml Thionylchlorid versetzt, anschließend während 90 Minuten gerührt und eingedampft. Zum Rückstand gibt man Toluol und dampft ein. Man erhält nach Umkristallisieren aus Methylenchlorid/Cyclohexan 5-Chlor-10,11-dihydro-1-methyl-5H-dibenzo[a,d]cyclohepten vom Schmelzpunkt 126—128°.

c) Eine Mischung aus 20,9 g Magnesiumspäne und 400 ml Äther wird mit wenig Jod versetzt, auf Siedetemperatur erwärmt und über einen Zeitraum von 30 Minuten tropfenweise mit einer Mischung aus 138 g Malonsäure-diäthylester und 200 ml trockenem Äthanol versetzt. Man erhitzt noch während 2 Stunden unter Rückfluß zum Sieden und dampft die erhaltene Suspension anschließend ein. Zum Rückstand gibt man 400 ml Benzol, dampft ein, löst ihn in 500 ml trockenem Tetrahydrofuran auf. Diese Lösung versetzt man bei 40° über einen Zeitraum von 20 Minuten mit einer Lösung von 190 g 5-Chlor-10,11-dihydro-1-methyl-5H-dibenzo[a,d]cyclohepten in 600 ml trockenem Tetrahydrofuran, erhitzt während 18 Stunden unter Rückfluß zum Sieden und engt das erhaltene Reaktionsgemisch ein. Der Rückstand wird zwischen Äther und Eiswasser verteilt; die organische Phase wird mit 1N Salzsäure, zweimal mit Eiswasser, einmal mit gesättigter Natriumbicarbonatlösung und mit Wasser gewaschen, über Magnesiumsulfat getrocknet und eingedampft. Man erhält rohen, kristallinen 10,11-Dihydro-1-methyl-5H-dibenzo[a,d]cyclohepten-5-malonsäure-diäthylester, das ohne weitere Reinigung in die nächste Stufe eingesetzt wird. Nach zweimaligem Umkristallisieren aus Diäthyläther erhält man reines Material vom Schmelzpunkt 79—80°.

d) Eine Mischung aus 294 g rohem 10,11-Dihydro-1-methyl-5H-dibenzo[a,d]cyclohepten-5-malonsäure-diäthylester und 1,5 l Äthanol wird mit 128 g Kaliumhydroxid in 400 ml Wasser vesetzt. Die erhaltene Mischung wird während 90 Minuten unter Rühren zum Sieden erhitzt und anschließend eingedampft. Der Rückstand wird zwischen 2 l Eiswasser und zweimal je 500 ml Chloroform verteilt. Die wäßrige Phase wird mit 500 ml Chloroform gewaschen und mit konzentrierter Salzsäure auf pH 2 gestellt. Der ausgefallene Festkörper wird abfiltriert, mit Wasser neutral gewaschen und im Vakuum bei 60° über Phosphorpentoxid getrocknet. Man erhält 10,11-Dihydro-1-methyl-5H-dibenzo[a,d]cyclohepten-5-malonsäure als beige Kristalle vom Schmelzpunkt 195—198°.

e) 176 g 10,11-Dihydro-1-methyl-5H-dibenzo[a,d]cyclohepten-5-malonsäure werden während 50 Minuten unter Rühren auf 170—175° erhitzt. Anschließend versetzt man mit 300 ml Benzol und erhitzt unter Rückfluß zum Sieden. Man filtriert, um unlösliche Anteile zu entfernen, und versetzt bei der

Siedetemperatur mit 600 ml Cyclohexan. Nach Abkühlen auf 10° wird das ausgefallene Material abfiltriert. Man erhält 10,11-Dihydro-1-methyl-5H-dibenzo[a,d]cyclohepten-5-essigsäure vom Schmelzpunkt 162—164°.

f) Eine Mischung aus 138 g 10,11-Dihydro-1-methyl-5H-dibenzo[a,d]cyclohepten-5-essigsäure, 100 ml Benzol und 308 g Thionylchlorid wird während 150 Minuten unter Rückfluß zum Sieden erhitzt und anschließend eingedampft. Den Rückstand versetzt man zweimal mit Benzol und dampft jeweils wieder ein. Man erhält 10,11-Dihydro-1-methyl-5H-dibenzo[a,d]cyclohepten-5-essigsäurechlorid, als rotbraunes Öl, das ohne weitere Reinigung in die nächste Stufe eingesetzt wird.

g) Eine Mischung aus 215 ml Pyrrolidin und 400 ml Benzol wird bei 0 bis 20° über einen Zeitraum von 45 Minuten tropfenweise mit einer Lösung von 147 g 10,11-Dihydro-1-methyl-5H-dibenzo[a,d]cyclohepten-5-essigsäurechlorid in 400 ml Benzol versetzt. Man rührt während 120 Minuten bei 40° und gießt auf Eiswasser. Die wäßrige Phase wird mit Benzol ausgeschüttelt. Die vereinigten organischen Auszüge werden nacheinander zweimal mit je 250 ml 2N Natronlauge, 250 ml Wasser, 250 ml 2N Salzsäure und gesättigter Kochsalzlösung gewaschen. Die organische Phase wird über Magnesiumsulfat getrocknet und eingedampft. Der Rückstand wird in 700 ml Petroläther (Siedebereich 80—105°) gelöst. Man kühlt die erhaltene Lösung auf 4° und filtriert das auskristallisierte Material ab. Man erhält 1-[(10,11-Dihydro-1-methyl-5H-dibenzo[a,d]cyclohepten-5-yl)acetyl]-pyrrolidin vom Schmelzpunkt 106—109°.

h) 18,6 g Lithiumaluminiumhydrid werden in 250 ml trockenem Dioxan vorgelegt. Dann gibt man bei der Siedetemperatur über einen Zeitraum von 60 Minuten tropfenweise eine Lösung von 156 g 1-[(10,11-Dihydro-1-methyl-5H-dibenzo[a,d]cyclohepten-5-yl)acetyl]pyrrolidin in 600 ml trockenem Dioxan hinzu. Nach 20 Minuten bei der Siedetemperatur versetzt man bei 10 bis 40° langsam mit 250 ml Essigsäureäthylester und anschließend bei 10° mit 150 ml Wasser. Nach Filtrieren über Kieselgur wird eingedampft und der Rückstand zwischen Äther und kalter 1N Salzsäure verteilt. Die organische Phase wird noch mit 2N Salzsäure ausgeschüttelt. Die vereinigten wäßrigen Auszüge werden unter Eiskühlung mit konzentrierter Natronlauge auf pH 10—12 gestellt und mit Methylenchlorid ausgeschüttelt. Die Methylenchloridphase wird mit Wasser neutral gewaschen, über Magnesiumsulfat getrocknet und eingedampft. Man erhält 1-[2-(10,11-Dihydro-1-methyl-5H-dibenzo[a,d]cyclohepten-5-yl)äthyl]pyrrolidin vom Schmelzpunkt 58—61°.

i) Eine Mischung aus 170 ml flüssigem Ammoniak, 6,1 g 1-[2-(10,11-Dihydro-1-methyl-5H-dibenzo[a,d]cyclohepten-5-yl)äthyl]pyrrolidin, 180 ml trockenem Tetrahydrofuran und 50 ml trockenem t-Butanol wird bei der Siedetemperatur und über einen Zeitraum von 30 Minuten mit 0,69 g Lithiumdraht versetzt. Nachdem die dunkelblaue Färbung auf farblos umgeschlagen hat, werden 20 ml Äthanol zugetropft und das Ammoniak abdestilliert. Der Rückstand wird mit Diäthyläther verdünnt und dreimal mit gesättigter Natriumchloridlösung gewaschen, über Magnesiumsulfat getrocknet und eingedampft. Man erhält 1-[2-(4,5,10,11-Tetrahydro-9-methyl-1H-dibenzo[a,d]cyclohepten-5-yl)äthyl]pyrrolidin als hellgelbes Öl. Das entsprechende Maleinsäuresalz hat nach zweimaligem Umkristallisieren aus Aceton/n-Hexan einen Schmelzpunkt von 118—120°.

## Beispiel A

4,5,10,11-Tetrahydro-N,N,9-trimethyl-1H-dibenzo[a,d]cyclohepten-5-propylamin kann wie folgt als Wirkstoff zur Herstellung von pharmazeutischen Präparaten verwendet werden:

| Kapseln | mg/Kapsel |
|---|---|
| Wirkstoff | 6,98 |
| Maisstärke | 20,00 |
| Milchzucker pulv. | 40,00 |
| Milchzucker krist. | 68,02 |
| Talk | 4,50 |
| Magnesiumstearat | 0,50 |
| Kapselfüllgewicht | 140,00 |

Der Wirkstoff wird mit den Hilfsstoffen gemischt und gesiebt. Nach erneutem Mischen wird die erhaltene Kapselfüllmasse auf einer vollautomatischen Kapselabfüllmaschine in Gelatinesteckkapseln geeigneter Größe abgefüllt.

**Patentansprüche**

1. Cycloheptenderivate der allgemeinen Formel

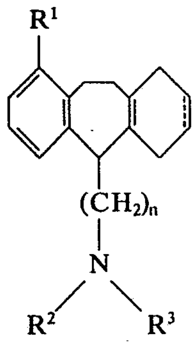

(I)

worin $R^1$ niederes Alkyl, die gestrichelte Linie eine fakultative Bindung und n die Zahl 1, 2 oder 3 bedeuten und entweder $R^2$ Wasserstoff oder niederes Alkyl und $R^3$ niederes Alkyl bedeuten, oder $R^2$ und $R^3$ zusammen mit dem Stickstoffatom einen 5- oder 6-gliedrigen Heterocyclus bedeuten, und pharmazeutisch annehmbare Säureadditionssalze davon.

2. Verbindungen gemäß Anspruch 1, dadurch gekennzeichnet, daß $R^1$ Methyl bedeutet.

3. Verbindungen gemäß Anspruch 1 oder 2, dadurch gekennzeichnet, daß der Rest $-NR^2R^3$ Methylamino, Dimethylamino, Diäthylamino, 1-Pyrrolidinyl oder 1-Piperidinyl bedeutet.

4. 4,5,10,11-Tetrahydro-N,N,9-trimethyl-1H-dibenzo[a,d]cyclohepten-5-propylamin.

5. 2,3,4,5,10,11-Hexahydro-N,N,9-trimethyl-1H-dibenzo[a,d]cyclohepten-5-propylamin.

6. 1-[2-(4,5,10,11-Tetrahydro-9-methyl-1H-dibenzo[a,d]cyclohepten-5-yl)äthyl]pyrrolidin.

7. Verbindungen gemäß einem der Ansprüche 1 bis 6 als pharmazeutische Wirkstoffe.

8. Verbindungen gemäß einem der Ansprüche 1 bis 6 als Histamin-$H_1$ antagonistische Wirkstoffe.

9. Verfahren zur Herstellung von Verbindungen gemäß einem der Ansprüche 1 bis 6, dadurch gekennzeichnet, daß man

a) in einer Verbindung der allgemeinen Formel

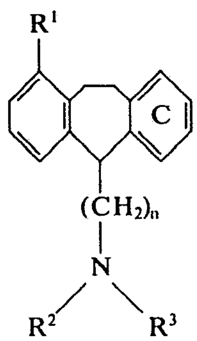

(II)

worin $R^1$, $R^2$, $R^3$ und n die in Anspruch 1 angegebene Bedeutung besitzen, den mit C bezeichneten aromatischen Ring 1,4-reduziert, oder

b) in einer Verbindung der allgemeinen Formel

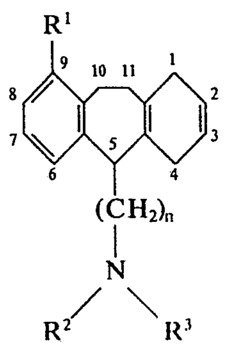

(Ia)

worin $R^1$, $R^2$, $R^3$ und n die in Anspruch 1 angegebene Bedeutung besitzen, die 2,3-Doppelbindung hydriert, oder

c) eine Verbindung der allgemeinen Formel

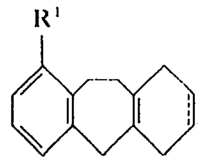

(III)

worin $R^1$ und die gestrichelte Linie die in Anspruch 1 angegebene Bedeutung besitzen, in Gegenwart einer starken Base mit einer Verbindung der allgemeinen Formel

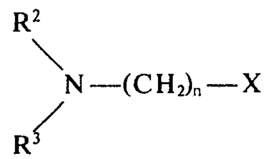

(IV)

worin $R^2$, $R^3$ und n die in Anspruch 1 angegebene Bedeutung besitzen, und X eine Abgangsgruppe bedeutet,
umsetzt, oder

d) eine Verbindung der allgemeinen Formel

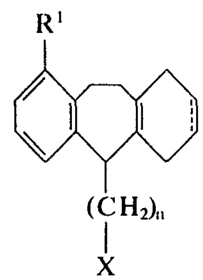

(V)

worin $R^1$, die gestrichelte Linie und n die in Anspruch 1 angegebene und X obige Bedeutung besitzen,
mit einem Amin der allgemeinen Formel

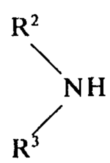

(VI)

worin $R^2$ und $R^3$ die in Anspruch 1 angegebene Bedeutung besitzen,
umsetzt, und

e) eine erhaltene Verbindung der allgemeinen Formel I als freie Base oder als pharmazeutisch annehmbares Säureadditionssalz isoliert.

10. Arzneimittel, enthaltend eine Verbindung gemäß einem der Ansprüche 1 bis 6.

11. Histamin-$H_1$ antagonistische Mittel, enthaltend eine Verbindung gemäß einem der Ansprüche 1 bis 6.

## Claims

1. Cycloheptene derivatives of the general formula

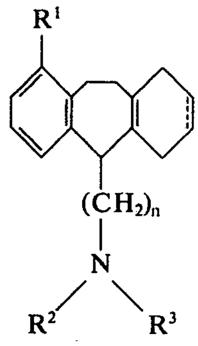

(I)

wherein $R^1$ signifies lower alkyl, the dotted line signifies an optional bond and n signifies the number 1, 2 or 3 and either $R^2$ signifies hydrogen or lower alkyl and $R^3$ signifies lower alkyl, or $R^2$ and $R^3$ together with the nitrogen atom signify a 5- or 6-membered heterocycle.
and pharmaceutically acceptable acid addition salts thereof.

2. Compounds in accordance with claim 1, characterized in that $R^1$ signifies methyl.

3. Compounds in accordance with claim 1 or 2, characterized in that the residue $-NR^2R^3$ signifies methylamino, diamethylamino, diethylamino, 1-pyrrolidinyl or 1-piperidinyl.

4. 4,5,10,11-Tetrahydro-N,N,9-trimethyl-1H-dibenzo[a,d]cycloheptene-5-propylamine.

5. 2,3,4,5,10,11-Hexahydro-N,N,9-trimethyl-1H-dibenzo[a,d]cycloheptene-5-propylamine.

6. 1-[2-(4,5,10,11-Tetrahydro-9-methyl-1H-dibenzo[a,d]cyclohepten-5-yl)ethyl]pyrrolidine.

7. Compounds in accordance with any one of claims 1 to 6 as pharmaceutically active substances.

8. Compounds in accordance with any of claims 1 to 6 as active substances which antagonize histamine-$H_1$.

9. A process for the manufacture of compounds in accordance with any one of claims 1 to 6, characterized by

a) 1,4-reducing the aromatic ring denoted by C in a compound of the general formula

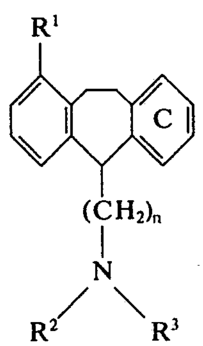

(II)

wherein $R^1$, $R^2$, $R^3$ and n have the significance given in claim 1,
or

b) hydrogenating the 2,3-double bond in a compound of the general formula

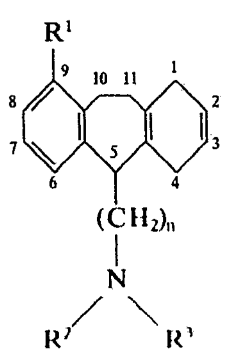

(Ia)

wherein $R^1$, $R^2$, $R^3$ and n have the significance given in claim 1,
or

c) reacting a compound of the general formula

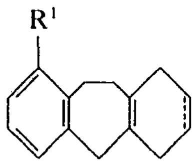

(III)

wherein $R^1$ and the dotted line have the significance given in claim 1,
in the presence of a strong base with a compound of general formula

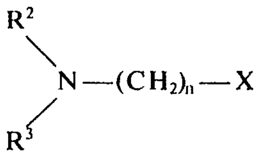

(IV)

wherein $R^2$, $R^3$ and n have the significance given in claim 1 and X signifies a leaving group,
or

d) reacting a compound of the general formula

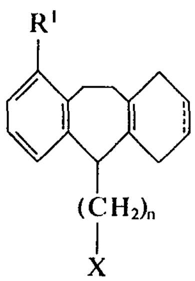

(V)

wherein $R^1$, the dotted line and n have the significance given in claim 1 and X has the above significance,
with an amine of the general formula

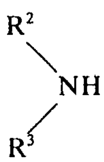

(VI)

wherein $R^2$ and $R^3$ have the significance given in claim 1,
and

e) isolating a compound of general formula I obtained as the free base or as a pharmaceutically acceptable acid addition salt.

10. Medicaments containing a compound in accordance with any one of claims 1 to 6.

11. Histamine-$H_1$-antagonistic medicaments containing a compound in accordance with any one of claims 1 to 6.

**Revendications**

1. Dérivés du cycloheptène de formule générale:

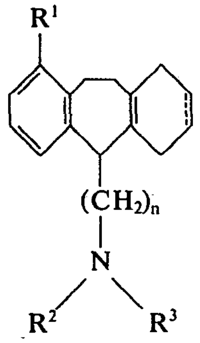

(I)

dans laquelle $R^1$ représente un groupe alkyle inférieur, le trait interrompu représente une liaison facultative et n est à 1, 2 ou 3, et ou bien $R^2$ représente l'hydrogène ou un groupe alkyle inférieur et $R^3$ un groupe alkyle inférieur, ou bien $R^2$ et $R^3$ forment ensemble et avec l'atome d'azote un hétérocycle à 5 ou 6 chaînons,
et leurs sels formés par addition avec des acides acceptables pour l'usage pharmaceutique.

2. Composés selon la revendication 1, caractérisés en ce que $R^1$ représente un groupe méthyle.

3. Composés selon la revendication 1 ou 2, caractérisés en ce que le reste $-NR^2R^3$ est un reste méthylamino, diméthylamino, diéthylamino, 1-pyrrolidinyle ou 1-pipéridinyle.

4. La 4,5,10,11-tétrahydro-N,N,9-triméthyl-1H-dibenzo[a,d]cycloheptène-5-propylamine.

5. La 2,3,4,5,10,11-hexahydro-N,N,9-triméthyl-1H-dibenzo[a,d]cycloheptène-5-propylamine.

6. La 1-[2-(4,5,10,11-tétrahydro-9-méthyl-1H-dibenzo[a,d]cycloheptène-5-yl)éthyl]pyrrolidine.

7. Composés selon l'une des revendications 1 à 6, en tant que substances actives pharmaceutiques.

8. Composés selon l'une des revendications 1 à 6, en tant que substances actives antagonistes de l'activité $H_1$ de l'histamine.

9. Procédé de préparation des composés selon l'une des revendications 1 à 6, caractérisé en ce que:

a) dans un composé de formule générale:

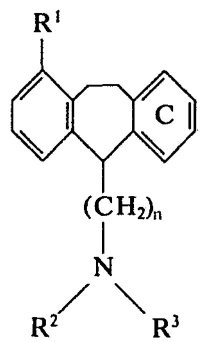

(II)

dans laquelle $R^1$, $R^2$, $R^3$ et n ont les significations indiquées dans la revendication 1, on soumet le cycle aromatique C à réduction en 1,4, ou bien

b) dans un composé de formule générale:

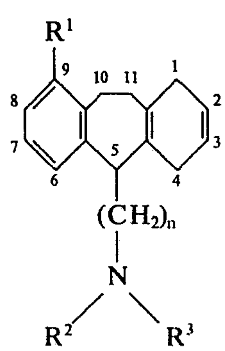

(Ia)

dans laquelle $R^1$, $R^2$, $R^3$ et n ont les significations indiquées dans la revendication 1, on hydrogène la double liaison en 2,3, ou bien

c) on fait réagir un composé de formule générale:

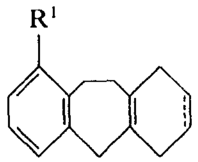

(III)

dans laquelle $R^1$ et le trait interrompu ont les significations indiquées dans la revendication 1, en présence d'une base forte, avec un composé de formule générale:

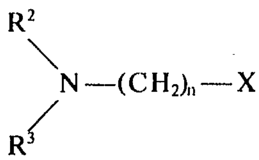

(IV)

dans laquelle $R^2$, $R^3$ et n ont les significations indiquées dans la revendication 1, et X représente un groupe éliminable, ou bien

d) on fait réagir un composé de formule générale:

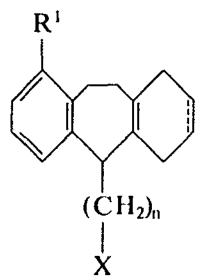

(V)

dans laquelle $R^1$, le trait interrompu et n ont les significations indiquées dans la revendication 1, et X a la signification indiquée ci-dessus, avec une amine de formule générale:

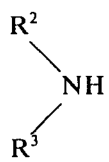

(VI)

dans laquelle $R^2$ et $R^3$ ont les significations indiquées dans la revendication 1, et

e) on isole un composé obtenu répondant à la formule générale I à l'état de base libre ou de sel formé par addition avec un acide acceptable pour l'usage pharmaceutique.

10. Médicaments contenant un composé selon l'une des revendications 1 à 6.

11. Agents antagonistes de l'activité $H_1$ de l'histamine, contenant un composé selon l'une des revendications 1 à 6.